# DEMANDE DE BREVET EUROPEEN

(11) **EP 2 348 012 A1**
(43) Date de publication de la demande: **27.07.2011**
(21) Numéro de dépôt: 10183123.8
(22) Date de dépôt: 09.04.2003
(51) Int. Cl.: C07C 213/02, C07D 263/06

(54) **PROCEDE DE PREPARATION DE COMBRETASTATINE**

(30) Priorité: 11.04.2002 FR 0204499
(62) Demande divisionnaire de: 03740582.6
(71) Demandeur: Aventis Pharma S.A., 92160 Antony (FR)
(72) Inventeur: Mutti, Stéphane, 75013, PARIS (FR); Lavigne, Michel, 75013, PARIS (FR); Malejonock, Irina, 75013, PARIS (FR); Casimir, Jean-Paul, 75013, PARIS (FR)
(74) Mandataire: Gaslonde, Aude

(57) **Abrégé**

La présente invention concerne de nouveaux procédés de préparation de combretastatines par condensation de Wittig entre le nitrométhoxy-benzaldéhyde et un sel de phosphonium du triméthoxybenzyle ou à l'inverse un sel de phosphonium du nitrométhoxybenzyle avec le triméthoxybenzaldéhyde.

## Description

La présente invention concerne un nouveau procédé de préparation de combrétastatines et de leurs dérivés.

On entend par combrétastatines ou encore dérivés du stilbène les dérivés de formule générale suivantes : 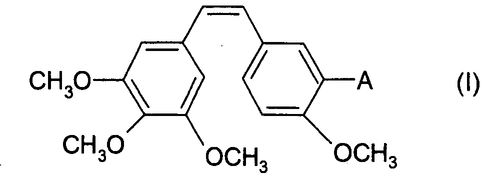 dans laquelle A représente un groupe hydroxyle ou un groupe amino ainsi que ses sels pharmaceutiquement acceptables.

Parmi les sels on peut citer le chlorhydrate, l'acétate, le phosphate, le méthanesulfonate. Quand le composé ou A est un groupe amino il peut aussi être couplé à des acides aminés pour conduire à des amides ainsi que leurs sels pharmaceutiquement acceptables.

La synthèse des dérivés du stilbène ou des combrétastatines qui peuvent être sous la forme d'un sel pharmaceutiquement acceptable ainsi que les compositions pharmaceutiques qui les contiennent sont décrites dans les brevets US 4,996,237 ; US 5,525,632; US 5,731,353 et US 5,674,906. Ces brevets décrivent les combrétastatines ainsi que leurs métabolites et décrivent leur activité ondologique in vitro.

Selon ces brevets les combrétastatines sont préparées à partir de sels de (3,4,5-triméthoxy-benzyle)-triphénylphosphonium qui est condensé sur un 3-nitro ou 3-hydroxy (dont le groupe hydroxyle est protégé) 4-méthoxy benzaldéhyde en présence d'hydrure de sodium ou de dérivés lithiés puis le dérivé obtenu, lorsqu'il est nitré est réduit en présence de zinc. L'isomère de configuration cis est ensuite préparé par action de la lumière ou par séparation chromatographique du mélange.

La présente invention concerne de nouveaux procédés de préparation de combrétastatines ou de leurs dérivés ainsi que des améliorations aux procédés existants.

Il a d'abord été découvert **un' premier procédé voie V0 1** de préparation de dérivés de formule (1) pour lesquels A représente un groupe amino, qui est une amélioration au procédé décrit dans les brevets cités précédemment qui consiste, après la condensation de Wittig en présence de bromure ou de chlorure de (3,4,5-triméthoxy-benzyle)-triphénylphosphonium et du 3-nitro 4-méthoxy benzaldéhyde, à réaliser la réduction en présence de fer, à la place du zinc utilisé dans l'antériorité, ce qui permet d'atteindre un rendement global de la réaction, par rapport à l'atdéhyde engagé, de 60 % (le rendement par rapport à l'aldéhyde engagé dans le brevet US 5,525,632 est compris entre 21 % à 33 %).

Le **premier procédé Voie V0 2** consiste à condenser le 3,4,5-triméthoxy-benzaldéhyde avec le bromure ou le chlorure du (4-méthoxy-3-nitro-benzyle)-triphénylphosphonium. Pour l'ensemble de ces deux premiers procédés voies V0 1 et V0 2, on opère en présence d'une base choisie parmi notamment le terbutylate de potassium, le teramylate de sodium, l'hydrure de sodium, le butyllithium, le LDA (Lithium diisopropylamine), le méthylate de sodium, le carbonate de potassium ou les derivés alcalins des hexaméthyldisilanes.

Cette réaction est mise en oeuvre dans des solvants divers comme les éthers (THF), les solvants aprotiques polaires (acétonitrile, NMP, DMF, DMSO.), les alcools, les solvants aromatiques ou l'eau, à une température qui sera adaptée par l'homme de l'art à la base utilisée et au solvant utilisé.

Cette réaction en ce qui concerne le premier procédé voie V0 2 est notamment décrite dans la publication de K.G. Piney parue dans Bioorg. Med. Chem. 8(2000) 2417-2425.

Le 2-méthoxy-5-[2-(3,4,5-triméthoxy-phényl)-vinyl]-nitrophényl est réduit selon le procédé amélioré de l'invention par l'action du fer. Il est avantageux d'utiliser une quantité de fer en excès si l'on veut une transformation complète du produit de départ. Cet excès est avantageusement supérieur à 2 équivalents pour une mole de dérivé nitré de départ.

Il a été prouvé, que la même étape réalisée en présence de zinc dans l'acide acétique, solvant classique des réductions au zinc, ne permet pas d'obtenir une réaction complète (dans le brevet US 5,525,632, le rendement de réduction réalisé sur l'isomère Z pur varie entre 46 et 66 %), et d'autre part les quantités de zinc utilisées sont énormes et conduisent par conséquence à des déchets industriels considérables, de plus le procédé génère une quantité de composé « azo » importante issu du couplage entre l'amino formé et l'intermédiaire nitroso de réduction.

La réduction à l'hydrogène naissant, généré par le formiate d'ammonium -en présence des catalyseurs classiques tels que le palladium ou le platine, entraîne une forte isomérisation de la double liaison en isomère E indésirable ainsi qu'une saturation partielle de la double liaison.

La publication de Piney préalablement citée décrit la réduction par l'hydrosulfite de sodium d'un isomère Z nitro pur, obtenu après chromatographie et recristallisation, conduisant à un isomère Z amino avec un rendement de seulement 37%.

Les hydrogénations à l'hydrogène moléculaire, catalysées par le platine ou le palladium sont rarement complètes et conduisent surtout à la saturation de la double liaison éthylénique.

Il a également été trouvé un **second procédé** évitant l'étape de réduction intermédiaire nécessaire lorsque l'on part d'un dérivé nitré. En effet il est beaucoup plus économique de condenser selon une première méthode de mise en oeuvre de ce second procédé un bromure ou un chlorure de (3,4,5-triméthoxy-benzyle)-triphénylphosphonium avec un 3-amino 4-méthoxy benzaldéhyde ou encore selon une deuxième méthode de mise en oeuvre de ce second procédé le 3,4,5-triméthoxybenzaldéhyde avec un sel du (3-amino-4-méthoxy-benzyle)- triphénylphosphonium.

Ce second procédé selon ses deux variantes nécessite une étape de moins engageant des produits CMR (Cancérogène, Mutagène, toxique pour la Reproduction), comparé au premiers procédés voies V0 1 et V0 2, ce qui au niveau industriel est un avantage du point de vue sécurité et coût de production considérable.

Selon **le second procédé voie V 03** de mise en oeuvre de l'invention, on met en présence le sel du (3,4,5-triméthoxy benzyle)-triphénylphosphonium avec le 3-amino-4-méthoxybenzaldéhyde, la réaction est mise en oeuvre, de préférence, en présence d'une base choisie parmi notamment le terbutylate de potassium, le teramylate de sodium, l'hydrure de sodium, le butyllithium, le LDA, le méthylate de sodium, le carbonate de potassium ou les derivés alcalins des hexaméthyldisilanes. On préfère utiliser le méthylate de sodium.

Cette réaction est mise en oeuvre dans des solvants divers comme les éthers (THF), les solvants aprotiques polaires (acétonitrile, NMP, DMF, DMSO..) les alcools, les solvants aromatiques ou l'eau, à une température qui sera adaptée par l'homme de l'art à la base utilisée et au solvant utilisé.

La température de réaction sera adaptée par l'homme de l'art à la base utilisée, lors de l'utilisation du méthylate, elle est de préférence comprise entre 0 et 10°C. Après réaction, la base utilisée est neutralisée par un acide en solution aqueuse, la phase organique est lavée, concentrée et le produit attendu est obtenu après chromatographie du concentrat brut.

**Selon le second procédé voie V0 4** de mise en oeuvre de l'invention, dans lequel on met en présence le sel du (3-amino-4-méthoxy benzyle)-triphénylphosphonium avec le 3,4,5-triméthoxy-benzaldéhyde, la réaction est, de préférence, mise en oeuvre en présence d'une base organique choisie parmi notamment le terbutylate de potassium, le teramylate de sodium, l'hydrure de sodium, le butyllithium, le LDA, le méthylate de sodium, le carbonate de potassium ou les derivés alcalins des hexaméthyldisilanes. On préfère utiliser le méthylate de sodium.

Cette réaction est mise en oeuvre dans des solvants divers comme les éthers (THF), les solvants aprotiques polaires (acétonitrile, NMP, DMF, DMSO..), les alcools, les solvants aromatiques ou l'eau, à une température qui sera adaptée par l'homme de l'art à la base utilisée et au solvant utilisé.

La température de réaction sera adaptée par l'homme de l'art à la base utilisée, lors de l'utilisation du méthylate, elle est de préférence comprise entre 0 et 10°C. Après réaction, la base utilisée est neutralisée par un acide en solution aqueuse, la phase organique est lavée, concentrée et le produit attendu est obtenu après chromatographie du concentrat brut.

Le dérivé obtenu selon le second procédé voie V0 3 ou V0 4 ou lors de la deuxième étape du premier procédé voie V0 1 ou V0 2 présente la formule suivante :

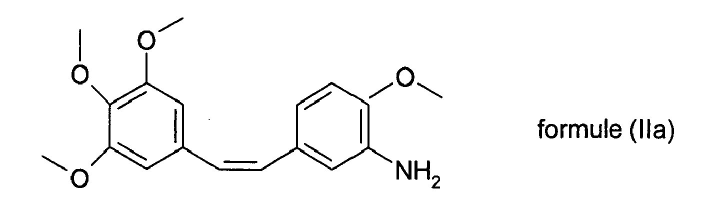

Il est avantageux, lorsque l'on cherche à coupler la sérine avec le composé de formule (Ila) d'engager de la L-sérine doublement protégée sur l'azote de la sérine et sur la fonction hydroxyle de formule générale (Ilb) 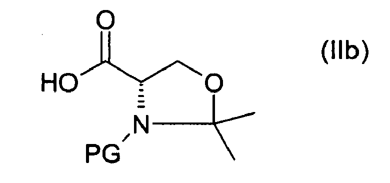 où PG représente un groupe protecteur de la fonction amine connu de tout homme de l'art pour donner un intermédiaire nouveau de formule générale suivante : 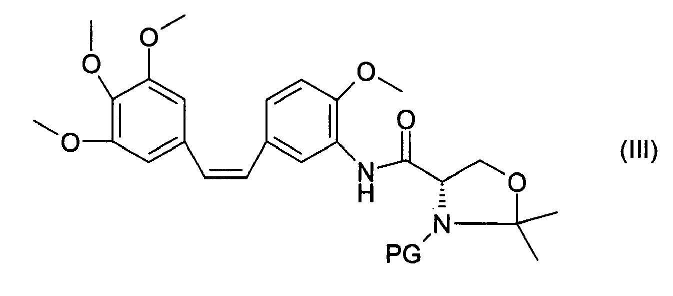 qui est ensuite clivé de préférence simultanément à l'ouverture du cycle par hydrolyse acide selon une réaction de déprotection connue de tout homme de l'art. De préférence le groupe PG des formules (Ilb) ou (III) représente un groupe protecteur choisi parmi les groupes suivants : ter-butoxycarbonyle, le benzyloxycarbonyle (CBZ) ou le 9-fluorenylmethyloxycarbonyl (FMOC).

Le composé de formule (III) ci-dessus est nouveau et est revendiqué en tant que tel.

La condensation est avantageusement réalisée en présence de EDCI (chlorure de 1-éthyl 3-(3-diméthylaminopropyl)carbodiimide) ou en présence de DCC (dicyclohexylcarbodiimide) et de HOBT (hydroxybenzotriazole) ou encore en présence de DCC (dicyclohexylcarbodiimide) et de HOSU (N-Hydroxy succinimide) ou enfin en présence de TOTU (O-[(Ethoxycarbonyl) cyanométhyièneamino]-N,N,N',N'-tetraméthyiuronium tetrafluoroborate ou de HBTU (0-Benzotriazol-1-yl-N,N,N',N'-tétraméthyluronium hexafluoro-phosphate) ou de N-N carbonyldiimidazole. Là réaction est de préférence mise en oeuvre dans un solvant inerte vis-à-vis de la réaction qui est choisi notamment parmi les solvants aprotiques polaires tels que l'acétonitrile, le diméthylformamide, le tétrahydrofurane ou les solvants aliphatiques chlorés tels que le dichlorométhane ou enfin les esters.

Le couplage sur le dérivé de formule (Ila) peut aussi bien être réalisé par l'action d'un anhydride mixte, synthétisé in-situ entre un chloroformiate ou un chlorure d'acide carboxylique par exemple de pivaloyle et la L-sérine doublement protégée de formule (Ilb) en présence d'une base tertiaire du type NMM (N-méthyl morpholine) dans des solvants divers inertes vis-à-vis de la réaction, esters, éthers, solvants chlorés, acétonitrile, etc.lanhydride mixte est préparé de préférence à une température comprise entre 0 et 10°C puis la réaction est réalisée à température ambiante. Après réaction on hydrolyse le milieu réactionnel avec une solution aqueuse puis on décante le milieu et la phase organique obtenue est lavée avec une base hydroxylée.

La double déprotection du composé de formule (III) est réalisée par l'action d'un acide organique ou inorganique, on préfère utiliser l'acide chlorhydrique concentré aqueux en milieu alcoolique La température de réaction est comprise selon un meilleur moyen de mise en oeuvre de l'invention entre 50 et 70°C.

L'invention sera plus complètement décrite à l'aide des exemples suivants qui ne doivent pas être considérés comme limitatifs de l'invention.

La composition des mélanges, le suivi et l'évolution des réactions ainsi que le rendement des produits/intermédiaires non isolés et leurs titres sont déterminés par analyse CLHP (Chromatographie Liquide Haute Performance).

### Exemple 1 - premier procédé voie V0 2 selon l'invention

Chlorhydrate du (Z)-N-[2-méthoxy-5-[2-(3,4,5-(triméthoxy-phényl) vinyl] phényl]-L-sérinamide

### Schéma général de la synthèse

Le nouveau procédé, dit «Wittig inverse» à partir de bromure de (4-méthoxy 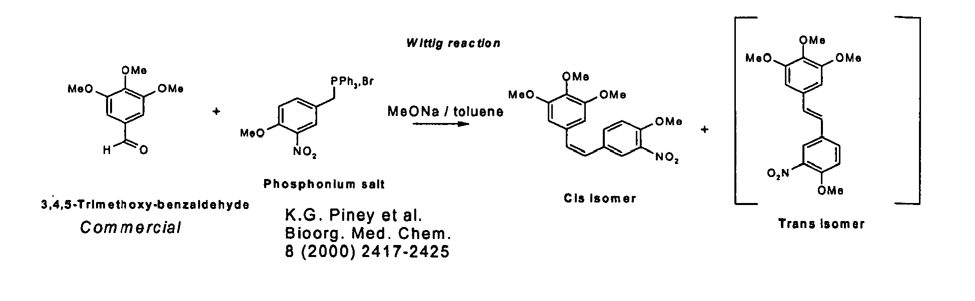 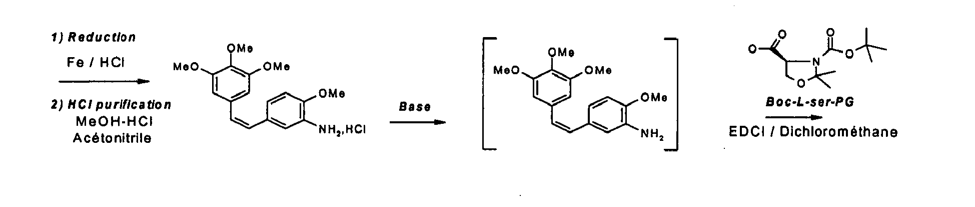 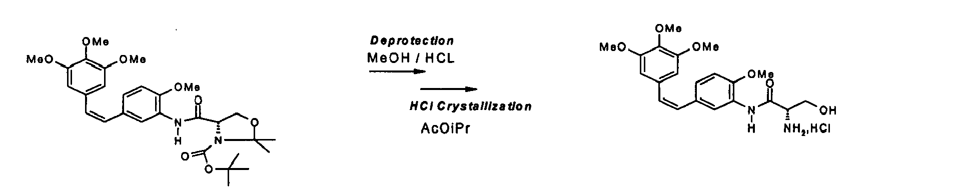 3-nitrobenzyl)-triphénylphosphonium et de 3,4,5-triméthoxy-benzaldéhyde permet d'obtenir le mélange d'isomères Z et E de 2-méthoxy-5-[2-(3,4,5-triméthoxy-phényl)-vinyl]-nitro-phényl avec un ratio Z/E de 75/25.

Ce ratio est suffisamment élevé en isomère nitro Z pour pouvoir engager directement le mélange Z/E en réduction et obtenir par cristallisation du chlorhydrate, l'isomère amino Z avec un titre CLHP de 97 % NI (normalisation interne).

La préparation du bromure du (4-méthoxy-3-nitro-benzyl)-triphénylphosphonium **(4)** est effectuée selon l'exemple suivant :

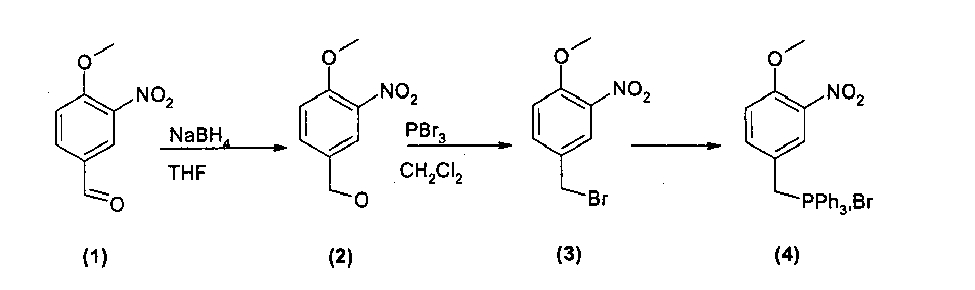

Alcool -3-nitro-4-méthoxy-benzylique **(2)** :

Dans un tricol de 2 litres muni d'une agitation mécanique, d'un thermomètre, d'un tube Y, d'un entonnoir d'addition pour solide et d'un réfrigérant surmonté d'un compte-bulles, 90,5 g de 3-nitro-4-méthoxy-benzaldéhyde **(1)** sont chargés, suivis de 450 ml de THF et de 90 ml d'éthanol. On refroidit la solution jaune pâle obtenue à 10°C, puis on charge 10 g de borohydrure de sodium en 40 minutes à 10/15°C (la réaction est très exothermique et la température doit être maintenue avec un bain de glace acétone), en fin d'addition la solution brune vire au bleu marine. La solution est agitée 30 minutes à 10°C, on contrôle la fin de réaction par CCM (chromatographie sur couche mince) et on agite encore 1 heure à 10°C puis on laisse la température revenir à l'ambiante.

L'entonnoir d'addition est remplacé par une ampoule de coulée isobare de 500 ml par laquelle on coule gouttes à gouttes 300 ml d'eau distillée en 30 minutes en maintenant le milieu à 20°C. On observe un dégagement gazeux en début de coulée.

Le milieu est concentré aux 2/3 à l'évaporateur rotatif (50°C/20 mmHg) un produit blanc cristallise dans le concentrat aqueux sous forme de blocs.

La phase aqueuse refroidie est extraite par 250 ml puis 150 ml de dichlorométhane, les phases organiques réunies sont lavées par 250 ml d'eau distillée, puis séchées sur sulfate de magnésium.

Après filtration la solution chlorométhylénique est engagée telle quelle dans la réaction de bromation suivante.

Le rendement de cette étape est considéré comme étant de 100 %.

NB : l'alcool **(2)** est commercial mais très peu disponible

3-nitro-4-méthoxy-bromobenzyle **(3)**:

Dans un tricol de 1 litre muni dune agitation mécanique, d'un thermomètre, d'un tube Y, d'une ampoule de coulée et d'un réfrigérant surmonté dun compte-bulles, on charge la solution chlorométhylénique d'alcool 3-nitro-4-méthoxy benzylique **(2)** et on ajoute 100 ml de dichlorométhane. La solution agitée est refroidie à 5°C puis on coule goutte à goutte en maintenant la température à 5°C, 135,4g de tribromure de phosphore.

La solution est agitée 1 heure 30 minutes à 5°C on contrôle la fin de réaction par CCM puis on coule goutte à goutte en maintenant la température à 15°C, 250 ml de solution saturée d'hydrogénocarbonate de sodium, il se produit un très fort dégagement gazeux avec léger retard pendant la coulée.

Le milieu décanté en ampoule est lavé successivement par 250 ml d'eau distillée et 200 ml de solution saturée d'hydrogénocarbonate de sodium. La phase organique est séchée sur sulfate de magnésium, filtrée et concentrée à l'évaporateur rotatif (50°C/20 mmHg).

On obtient 119 g de solide sous forme d'aiguilles feutrées jaune-vert avec un rendement chimique sur 2 étapes de 97 %.

NB : Ce produit (3) peut aussi être préparé selon le schéma suivant décrit dans la publication K. G. Piney et al. Bioorg. Med. Chem. 8 (2000) 2417-2425

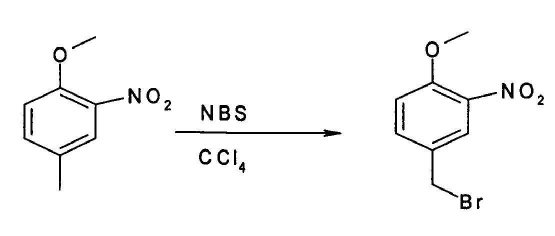

Bromure de (3-nitro-4-méthoxy-benzyl)-triphénylphosphonium **(4)**:

Dans un tricol de 2 litres, muni d'une agitation mécanique, d'un thermomètre, d'un tube Y, d'un entonnoir d'addition pour solide et d'un réfrigérant surmonté d'un compte-bulles, 119 g de 3-nitro-4-méthoxy-bromobenzyle **(3)** sont chargés sur un pied agité de 1000 ml de toluène, le milieu tiédi à 25°C passe en solution. On ajoute alors 126,5 g de triphénylphosphine, la solution obtenue est chauffée progressivement à 60°C, dès 30°C il se forme un précipité. Le milieu est maintenu 4 heures à 60/65°C puis refroidi à 30°C, filtré sur verre fritté, lavé sur filtre par 2 fois 300 ml de toluène, essoré et séché à l'étuve (35°C/20 mmHg/20 heures).

On obtient 217g de bromure de (4-méthoxy-3-nitro-benzyl)-triphénylphosphonium avec un rendement chimique de 88 %.

Synthèse décrite dans la publication : (solvant utilisé : dichlorométhane)
K. G. Piney et al. Bioorg. Med. Chem. 8 (2000) 2417-2425

Spectre n° = 4 865 - V
Spectre de R.M.N. ¹H (300 MHz, (CD₃)₂SO d6, δ en ppm) : 3,90 (s : 3H) ; 5,26 (d, J = 15 Hz : 2H) ; 7,33 (mt : 2H) ; 7,41 (mt : 1H) ; de 7,65 à 8,05 (mt : 15H).

Spectre de masse n°212217, m=428
El m/z=262 [PPh₃]⁺ pic de base

DCI
m/z=445 MNH₃⁺
m/z=428 M⁺
m/z=263 [PPh₃H]⁺ pic de base

Spectre IR 426469 KBr
2869; 2843; 2776; 1619; 1527; 1438; 1362; 1287; 1270; 1111; 752; 692 et 502 cm⁻¹

Mélange Z et E de 2-méthoxy-5-[2-(3,4,5-triméthoxy-phényl)-vinyl]-nitrophényl (6) et (7) :

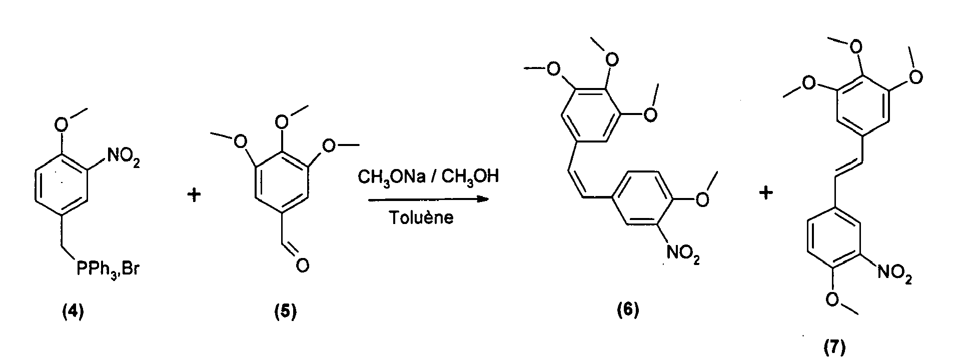

Dans un tricol de 2 litres, muni d'une agitation mécanique, d'un thermomètre, d'un tube Y, dune ampoule de coulée et dun réfrigérant surmonté dun compte-bulles, on charge à 20°C et sous azote, 54,7g de 3,4,5-triméthoxybenzaldéhyde **(5)**, 148,6g de bromure du (4-méthoxy-3-nitrobenzyl)-triphénylphosphonium **(4)** et 1300 ml de toluène.

La suspension agitée est refroidie à 5°C à l'aide d'un bain de glace, puis on coule à 5°C en 40 minutes, 63,2 g d'une solution de méthylate de sodium à 25 % p/p dans le méthanol.

Au fur et à mesure de la coulée la suspension passe du blanc cassé au jaune puis au brun.

On agite 1 heure à 5°C, la fin de réaction est contrôlée par CLHP (consommation totale de l'aldéhyde), 3 g (0,05 mole) d'acide acétique sont alors ajoutés.

La suspension est chauffée à 40°C et maintenue 30 minutes à 40°C, à cette température, seuls les sels restent insolubles, on filtre à 40°C sur verre fritté N°3 et on lave les sels sur filtre par 3 fois 100 ml de toluène.

Le filtrat est rechargé en ballon avec 250 ml d'eau distillée, le mélange bi-phasique est agité 20 minutes à 40°C puis décanté en ampoule. La phase toluénique est lavée par encore 2 fois 250 ml d'eau distillée puis concentrée à sec à l'évaporateur rotatif.

Le culot est repris par 600 ml d'isopropanol et 12 ml de toluène à 40°C, l'attendu commence à cristalliser, on laisse sous agitation lente la température revenir à l'ambiante jusqu'au lendemain.

La suspension agitée est refroidie et maintenue 1 heure à 5°C puis filtrée sur verre fritté, le gâteau est lavé par 2 fois 125 ml d'isopropanol, essoré et séché à l'étuve sous vide (35°C / 30mmHg / 18 heures).

On obtient 91,7 g d'un mélange d'isomères Z et E **(6)** et **(7)** avec un ratio Z/E de 75/25 (CLHP NI) et un rendement de 95 %.

Synthèse décrite dans la publication : (solvant utilisé : dichlorométhane : base utilisée : NaH)
K. G. Piney et al. Bioorg. Med. Chem. 8 (2000) 24 7-2425

NB : De nombreuses conditions opératoires ont été expérimentées telle que :
Solvants : THF, acétonitrile, méthanol et autres alcools, dichlorométhane, NMP, DMF, DMSO, etc...
Bases : t-butylate de potassium, t-amylate de sodium, soude, NaH, Buli/LDA, carbonate de potassium, etc.

Températures : de-10°C au reflux de certains solvants

Chlodhydrate du Z-2-méthoxy-5-[2-(3,4,5-triméthoxyphényl)-vinyl]-phénylamine **(8)** :

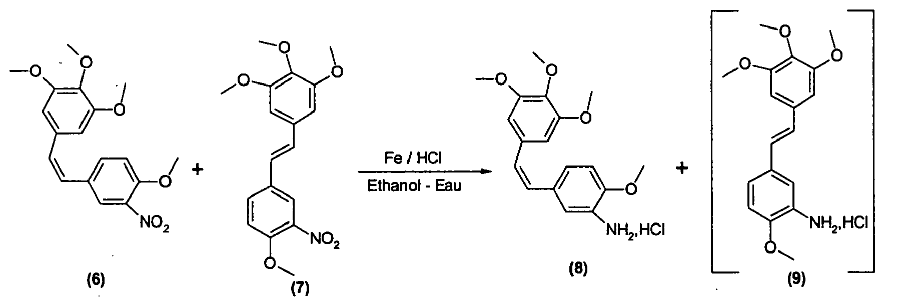

Dans un tricol de 2 litres muni dune agitation mécanique, d'un thermomètre, d'un tube Y, d'un entonnoir d'addition pour solide, dun réfrigérant surmonté dun compte-bulles et d'un bain chauffant, on charge à 20°C et sous azote 80 g de mélange Z et E 75 / 25 de 2-méthoxy-5-[2-(3,4,5-triméthoxy-phényl)-vinyl]-nitro-phényl **(6) et (7),** 640 ml d'éthanol absolu et 160 ml d'eau distillée.

On agite rapidement et on chauffe au bain d'huile, à 50°C on ajoute à la suspension 7,8 ml d'acide chlorhydrique 6N puis on monte la température du milieu à 77 ± 2°C, le milieu est presque soluble.

On ajoute en 5 minutes par fractions, 52 g de fer en poudre. Dès le premier ajout le milieu passe en solution puis un dépôt noirâtre se forme sur les parois du ballon.

On maintient 2 heures à 77 ± 2°C, on contrôle par CLHP la disparition des nitros **(6)** et **(7)** de départ.

On laisse refroidir à 40°C et on filtre le milieu sur verre fritté garni de clarcel, le gâteau est rincé par 2 fois 160 ml de mélange éthanol/eau 80/20.

Le filtrat, eaux mères et eaux de lavage, est concentré à l'évaporateur rotatif, dès que l'azéotrope a été chassé une huile commence à précipiter dans la phase aqueuse résiduelle.

On extrait cette phase aqueuse en ampoule par 2 fois 300 ml de dichlorométhane puis on lave la phase organique par 2 fois 300 ml de solution aqueuse demi-saturée de chlorure de sodium et par 300 ml d'eau distillée.

La phase organique est concentrée à sec à l'évaporateur rotatif, on obtient 76 g d'une huile qui présente un ratio Z/E de 80/20 en CLHP. Cette huile est dissoute dans 591 ml de méthanol et transvasée dans un ballon de 1 litre agité, on ajoute alors 100 ml de méthanol chlorhydrique 2,32N, on amorce et on laisse précipiter la nuit sous agitation.

La quantité de méthanol + méthanol chlorhydrique est telle que la concentration finale en isomère Z (déterminée par CLHP) soit égale à 8,8 % p/v.

Le lendemain le milieu est filtré sur verre fritté, le gâteau séché pèse 8,2 g et n'est composé que d'isomère E (CLHP).

Le filtrat (693 g) ratio Z/E = 86/14 (CLHP NI) est concentré au demi à l'évaporateur rotatif, on ajoute aux 347 g de concentrat, 400 ml d'acétonitrile et on reconcentre jusqu'à obtenir à nouveau un concentrat de 347 g. On ajoute alors 1000 ml d'acétonitrile et on concentre jusqu'à début de cristallisation, le concentrat est alors transvasé dans un ballon de 4 litres agité contenant 1500 ml d'acétonitrile à 60°C le milieu précipite abondamment.

On maintient 2,5 heures à 60°C sous agitation, on laisse refroidir à 30°C en 1 heure environ, la bouillie est filtrée sur verre fritté, l'isomère E **(9)** est soluble dans le filtrat, le gâteau est lavé par 2 fois 200 ml d'acétonitrile et séché à l'étuve (35°C / 30 mmHg / 18 heures).

On obtient 45,7 g de Z- 2-méthoxy-5-[2-(3,4,5-triméthoxy-phényl)-vinyl]-phénylamine **(8)** avec un titre CLHP Nl de 97 % et un rendement tel quel de 56 % soit un rendement en isomère Z obtenu sur isomère Z engagé de 72 %.

### EXEMPLE 2 - Synthèse selon le second procédé voie V0 3 selon l'invention

L'intérêt du second procédé voie V0 3 par rapport au premier procédé voie V0 2 dit de «Wittig inverse» est de réaliser la réaction de Wittig entre un produit déjà réduit, l'amino aldéhyde **(1a)** et le phosphonium **(2a)** et donc de supprimer une étape chimique engageant des produits CMR.

Chlorhydrate du (Z)-N-[2-méthoxy-5-[2-(3,4,5-(triméthoxy-phényl) vinyl] phényl]-L-sérinamide

Schéma général de la synthèse

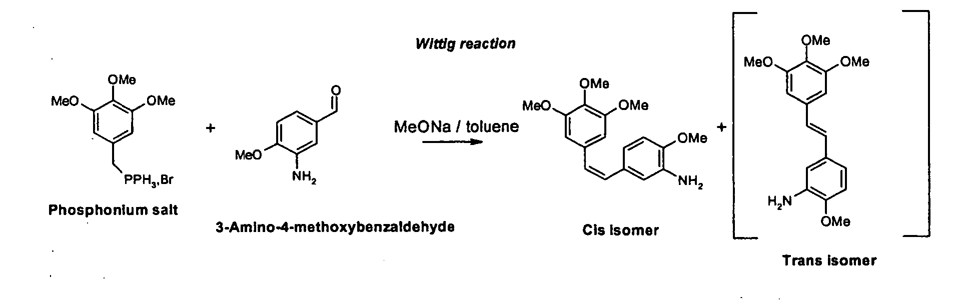

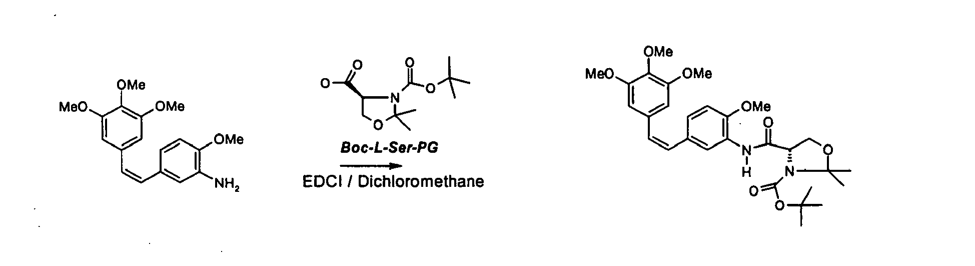

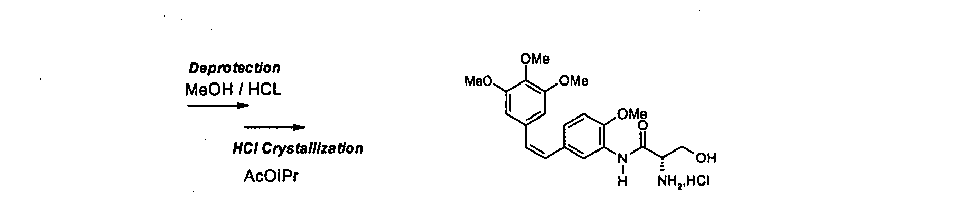

3-Amino-4-méthoxy-benzaldéhyde (1 a) :

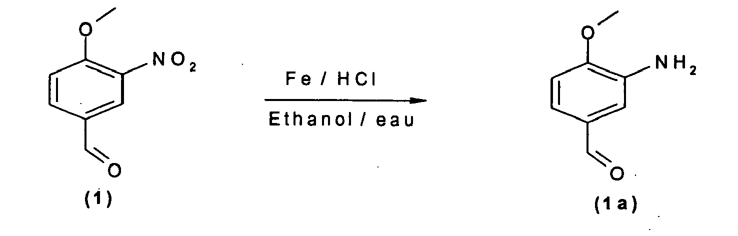

Dans un tricol de 2 litres inerté à l'argon et muni d'une agitation mécanique, d'un thermomètre, d'un tube Y, d'un entonnoir d'addition pour solide, d'un réfrigérant surmonté d'un compte-bulles et d'un bain chauffant, on charge 20 g de 3-nitro-4-méthoxy-benzaldéhyde **(1)** et 350 ml d'éthanol absolu, on agite, on chauffe à 60°C, le milieu passe en solution.

A 60°C, on ajoute goutte à goutte 115 ml d'eau distillée puis 14 ml d'acide chlorhydrique 2N. On introduit alors par petites fractions 24,7 g de fer en poudre.

On laisse la température du milieu revenir à l'ambiante en 2 heures. La réaction est complète (CCM).

Le milieu est filtré sur célite et concentré sous vide, le culot est repris au dichlorométhane et la solution organique est lavée 2 fois à l'eau distillée puis séchée sur sulfate de magnésium, filtrée et concentrée à sec sous vide.

On obtient 16 g de **(1a)** brut qui sont chromatographiés sur colonne de silice éluée au dichlorométhane.

On obtient 2 fractions contenant l'attendu propre qui après concentration délivrent 11,5 g de **(1a)** pur, soit un rendement de 69 %.

Spectre n°2810-V de R.M.N. ¹H (300 MHz, (CD₃)₂SO d6, δ en ppm) : 3,88 (s : 3H) ; 5,11 (mf : 2H) ; 7,01 (d, J = 8 Hz : 1 H) ; 7,14 (d, J = 2 Hz : 1H); 7,18 (d, J = 8 Hz : 1H) ; 9,53 (s : 1H).

Spectre de masse n°210112, m=151
El
m/z=151 M⁺ pic de base
m/z=136 [M-CH₃]⁺
m/z=108 [136-CO]⁺
m/z=80 [108-CO]⁺

Spectre IR: 425135 KBr
3464; 3437; 3367; 3349; 1675; 1655; 1582; 1513; 1293; 1241; 1139; 1023; 803 et 640 cm⁻¹

Z et E -2-méthoxy-5-[2-(3,4,5-triméthoxy-phényl)-vinyl]-phénylamine (8') et **(9')** :

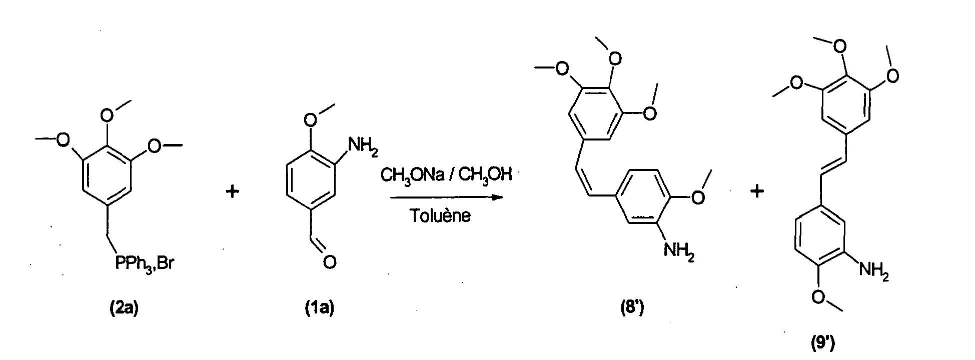

NB : Le phosphonium **(2a)** est une matière première déjà décrite dans le brevet original Ajinomoto Co., Ltd US 5,525,632 et WO 01/12579 A2.

Dans un tricol de 250 ml inerté à l'azote et muni d'une agitation magnétique, d'un thermomètre, dun tube Y, dune ampoule de coulée, d'un réfrigérant surmonté d'un compte-bulles et d'un bain réfrigérant, on charge 8,0 g de phosphonium **(2a)** puis 2,20 g damino benzaldéhyde **(1a)** et 100 ml de toluène. La suspension agitée est refroidie à 5°C et on coule en 15 minutes 3,51 ml de méthylate de sodium en solution méthanolique à 25 % p/p. Après 2,5 heures à 5°C la réaction reste incomplète (TT : 45 %) mais n'évolue plus (CLHP) et le ratio Z/E est de 61/39. On coule alors 0,2 ml d'acide acétique dilué dans 50 ml d'eau la température monte à 13°C on agite 30 minutes puis on décante en ampoule, la phase organique est concentrée sous vide au rotavapor et on obtient 8 g d'huile jaune.

En CLHP cette huile contient de l'aldéhyde de départ de la phosphine oxyde et le mélange Z / E attendu avec un ratio de 61/ 39.

L'huile est chromatographiée sur colonne de silice (40 parties p/p) éluée par un mélange de cyclohexane / acétate d'éthyle/ triéthylamine (50/50/2).

2 séries de fractions réunies sont concentrées à sec, le premier extrait sec de 360 mg contient l'isomère Z à 93 % + impuretés non identifiées, le second de 2,09 g contient de l'aldéhyde de départ et un mélange Z/E représentant 39 et 37,5 % en CLHP Nl.

Le bilan pondéral en isomère Z **(8')** déterminé par CLHP NI est de 1,15 g sur 2,20 g d'aldéhyde engagé soit un rendement de 24 %.

### EXEMPLE 3 - Synthèse selon le second procédé voie V0 3 selon l'invention

Comme pour la voie V0 2, l'intérêt de la voie V0 3 par rapport au premier procédé voie V0 2 dit de «Wittig inverse» est de réaliser la réaction de Wittig entre un produit déjà réduit, le bromure de (3-amino-4-méthoxy-benzyle)-triphénylphosphonium **(1b)** et le 3,4,5-triméthoxy benzaldéhyde (5) et donc de supprimer une étape chimique engageant des produits CMR.

Chlorhydrate du (Z)-N-[2-méthoxy-5-[2-(3,4,5-(triméthoxy-phényl) vinyl] phényl]-L-sérinamide

Schéma général de la synthèse

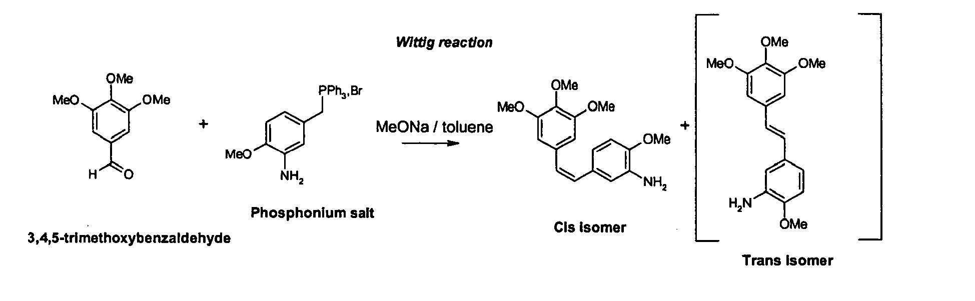

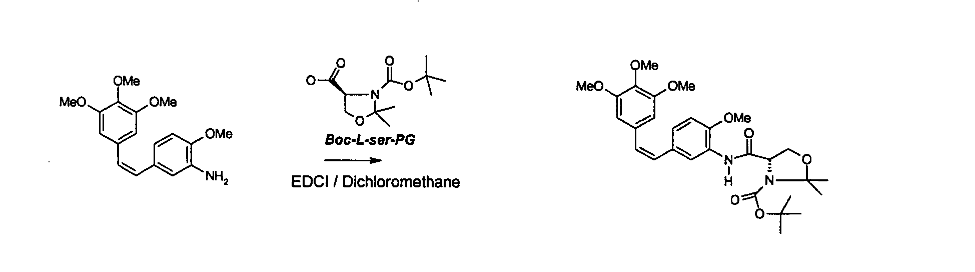

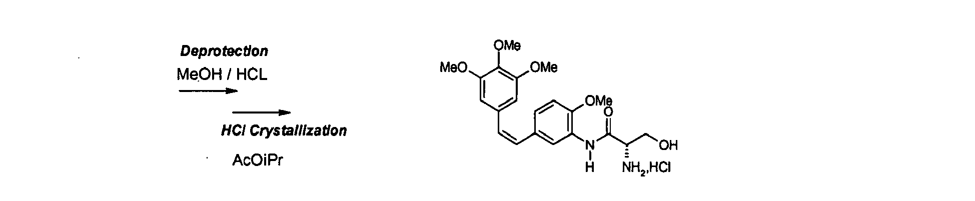

Bromure de (4-méthoxy-3-amino-benzyle)-triphénylphosphonium **(1b)**

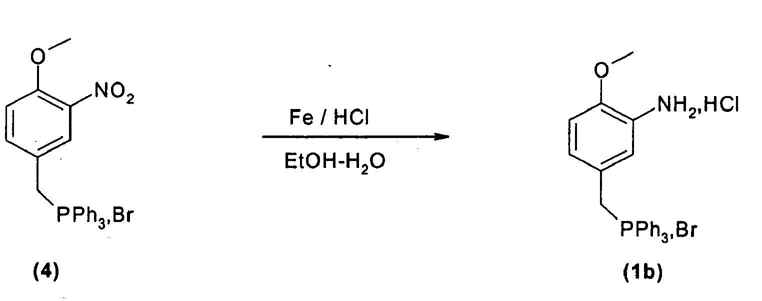

Dans un tricol d'1 litre muni dune agitation mécanique, d'un thermomètre, d'un tube Y, d'un entonnoir daddition pour solide, d'un réfrigérant surmonté d'un compte-bulles et d'un bain chauffant, on charge 30 g de **(4)**, 240 ml d'éthanol et 60 ml d'eau distillée.

Sur la suspension agitée, on ajoute 1,76 ml d'acide chlorhydrique 6N et on chauffe à 70°C.

On additionne alors en 15 minutes par petites fractions, 9,9 g de fer en poudre le milieu reste insoluble. Le milieu est maintenu 2 heures à 75°C, les organiques passent lentement en solution tandis qu'un dépôt brunâtre de fer et d'oxyde de fer se forme.

Après contrôle CLHP il reste encore 5 % de départ, on ajoute à nouveau 2 g de fer et on poursuit le chauffage pendant 1 heure, le TT est complet.

On refroidit à 40°C le milieu est filtré sur clarcel, rincé par 100 ml d'éthanol à 20 % d'eau et le filtrat est concentré à sec sous vide au rotavapor.

Le culot est repris par 300 ml d'isopropanol et cristallise dans le milieu, on agite et on chauffe à 50°C et le milieu repasse en solution. On coule alors 14 ml disopropanol chlorhydrique 5N, le milieu précipite, on maintient 1 heure à 50°C puis on laisse revenir à l'ambiante.

La bouillie est filtrée sur verre frittée, le gâteau est lavé par 50 ml d'isopropanol, essoré à fond et séché à l'étuve sous vide.

On obtient 27,3 g de **(1b)** avec un rendement tel quel de 89,9 %.

Spectre n° 4584-V de R.M.N. ¹H (300 MHz, (CD₃)₂SO d6, δ en ppm) : 3,78 (s : 3H) ; 5,03 (d large, J = 15 Hz : 2H) ; 6,43 (mf : 1 H) ; 6,62 (s large : 1H) ; 6,82 (d large, J = 8 Hz : 1 H) ; de 7,60 à 8,00 (mt : 15H).

Spectre de masse n°211915, m=397
El
   m/z=397 m⁺
   m/z=382 [M - CH₃]⁺
   m/z=262 [PPh₃]⁺ pic de base
DCI
   m/z=398 MNH₄⁺
   m/z=263 [PPh₃H]⁺ pic de base

Spectre IR 426386 KBr
3254; 2474; 1920; 1628; 1520; 1439; 1433; 1279; 1110; 736; 690; 527 et 511 cm⁻¹

Z et E -2-méthoxy-5-[2-(3,4,5-triméthoxy-phényl)-vinyl]-phénylamine (8') et **(9')**

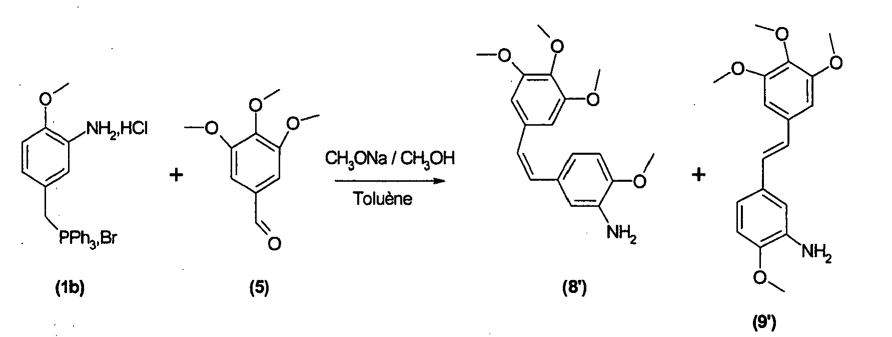

Dans un tricol de 250 ml inerté à l'azote et muni d'une agitation magnétique, d'un thermomètre, d'un tube Y, d'une ampoule de coulée, d'un réfrigérant surmonté d'un compte-bulles et d'un bain réfrigérant, on charge 11,02 g de **(1b),** 4 g de **(5)** et 70 ml de toluène.

La suspension agitée est refroidie à 5°C et on coule en 15 minutes, 4,92 ml de solution de méthylate de sodium dans le méthanol à 25 % p/p. On agite la suspension 2,5 heures à 5°C puis on coule alors 0,2 ml d'acide acétique dilué dans 50 ml d'eau, la température monte à 14°C et le milieu devient très épais, on dilue avec 10 ml de toluène et 10 ml d'eau, il reste un insoluble brun.

Le mélange est filtré sur clarcel, le gâteau est lavé par 3 fois 50 ml de toluène (les lavages ne contiennent pratiquement que de l'aldéhyde de départ et ne sont pas jointes au filtrat bi-phasique), le filtrat limpide (pH 12) est décanté en ampoule et la phase organique est concentrée à sec sous vide à 40°C le ratio Z/E déterminé par CLHP NI est de 43/57.

L'huile brune obtenue (4 g) est chromatographiée sur colonne de silice (100 parties p/p) éluée par un mélange de cyclohexane/ acétate d'éthyle/triéthylamine (50/50/2).
2 séries de fractions réunies sont concentrées à sec, le premier extrait sec de 1,1 g contient 14 % d'isomère E et 59 % de Z, le second pèse 1,08 g et contient 86 % d'isomère E et 7 % de Z.

Le bilan pondéral en isomère Z **(8')** déterminé par CLHP NI est de 0,725 g sur 4 g d'aldéhyde engagé soit un rendement tel quel de 11,3 %.

Z-4-{2-méthoxy-5-[2-(3,4,5-triméthoxy-phényl)-vinyl]-phénylcarbamoyl}-2,2-diméthyl-oxazolidine-3-carboxylic acid tert-butyl ester **(11)** :

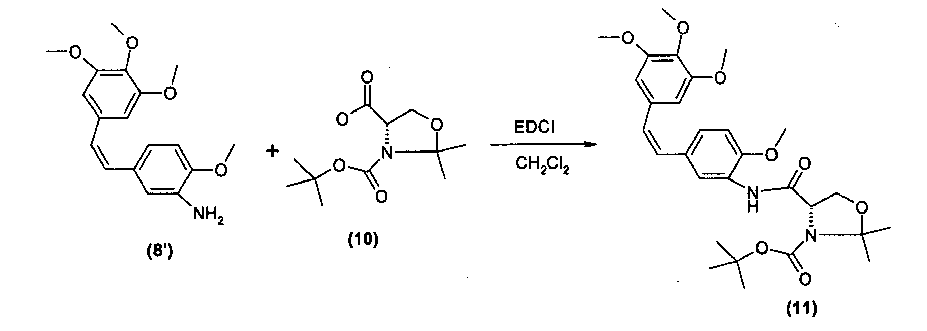

Libération de la base **(8')** à partir du chlorhydrate **(8)** :

Dans un erlenmayer de 1 litre on charge 44 g de (8), 16 g d'hydrogénocarbonate de sodium puis 200 ml deau distillée et 375 ml de dichlorométhane. On agite 20 minutes à l'ambiante, on obtient deux phases limpides.

La phase organique est séparée par décantation, séchée sur sulfate de sodium puis filtrée.

On obtient environ 400 ml de solution chlorométhylénique contenant (8').

Obtention du 2,2-diméthyl-oxazolidine-3,4-dicarboxylic acid-3-ter-butyl ester (10)

Ce produit est bien que commercial est très peu disponible, il a donc été préparé par saponification à la lithine de son ester méthylique selon selon : J. ORG. CHEM. 63 (12) P. 3983 (1998).

Spectre de R.M.N. ¹H (300 MHz, (CD₃)₂SO d6, δ en ppm) : 1,38 (s : 3H) ; 1,45 (s : 9H) ; 1,55 (s : 3H) ; 3,95 (mt : 1H); 4,16 (mt : 1H) ; 4,31 (mt : 1H) ; de 12,50 à 13,10 (mf étalé : 1H).

Spectre de masse:_n°213135, m=245
DCI
   m/z=263 MNH₄⁺
   m/z=246 MH⁺
   m/z=207 [MNH₄ - tBu]⁺ pic de base
   m/z=146 [MH - BOC]⁺

Spectre IR : 426759 KBr
1744; 1704; 1638; 1407; 1368; 1164; 1104; 856; 836 et 623 cm⁻¹

Couplage :

Dans un tricol de 2 litres muni d'une agitation mécanique, d'un thermomètre, dun tube Y, d'un entonnoir daddition pour solide, d'un réfrigérant surmonté d'un compte-bulles et d'un bain de glace, on charge la solution de (8'), on ajoute 600 ml de dichlorométhane et on refroidit sous agitation.

A 5°C on additionne 42,9 g de 2,2-diméthyl-oxazolidine-3,4-dicarboxylic acid-3-ter-butyl ester (10) qui passent en solution puis on ajoute par fractions entre 5 et 10°C, 48 g de 1-éthyl 3-(3-diméthylaminopropyl)carbodiimide, hydrochloride (EDCI).

On laisse lentement le milieu revenir à l'ambiante en laissant fondre la glace du bain au cours de la nuit.

Le lendemain, on ajoute 330 ml d'eau distillée et on agite vigoureusement. En 30 minutes le milieu se trouble (hydrolyse de l'EDCI) on maintient l'agitation pendant encore 30 minutes.

Le milieu est décanté en ampoule et la phase organique est lavée successivement par 2 fois 280 ml de soude 0,55 N puis par 300 ml d'eau distillée.

La phase organique est concentrée à sec à l'évaporateur rotatif (50°C / 50 mmHg)

On obtient 79,4 g dune huile collante (11) qui durcit à 20°C avec un rendement pondéral sur (8) engagé de 117 %.

Spectre n° = 5 578 - V

Spectre de R.M.N. ¹H (400 MHz, (CD₃)₂SO d6, à une température de 373 K, δ en ppm) : 1,41 (s : 9H) ; 1,53 (s : 3H) ; 1,64 (s : 3H) ; 3,64 (s : 6H) ; 3,71 (s : 3H) ; 3,86 (s : 3H) ; 3,99 (dd, J = 9 et 3Hz : 1H) ; 4,19 (dd, J = 9 et 7 Hz : 1H) ; 4,52 (dd, J = 7 et 3 Hz : 1H) ; 6,48 (d, J = 12,5 Hz : 1H) ; 6,55 (d, J = 12,5 Hz : 1H) ; 6,58 (s : 2H) ; 7,02 (mt : 2H) ; 8,13 (s large : 1H) ; 8,82 (s large : 1H).

Spectre de masse n°213565, m=542
DCI
   m/z=560 MNH₄⁺ pic de base
   m/z=543 MH⁺
   m/z=504 [MNH₄ - tBu]⁺
   m/z=443 [MH - BOC]⁺

Spectre IR 425857 CCI₄
3409; 2982; 2938; 2837; 1712; 1698; 1534; 1363; 1249; 1133; 1092 et 851 cm⁻¹

D'autres conditions de couplage ont été mises en oeuvre telles que:
- Anhydride mixte (chlorure de pivaloyle / (10))
- DCC / HOBT - DCC / HOSU - TOTU-N-N carbonyldiimidazole - etc..
- Dans Acétonitrile, DMF, THF, Diclorométhane, ester, etc.

L'EDCI, HCI dans le dichlorométhane a donné le meilleur résultat.

Chlorhydrate du (Z)-N-[2-méthoxy-5-[2-(3,4,5-(triméthoxy-phényl) vinyl] phényl]-L-sérinamide

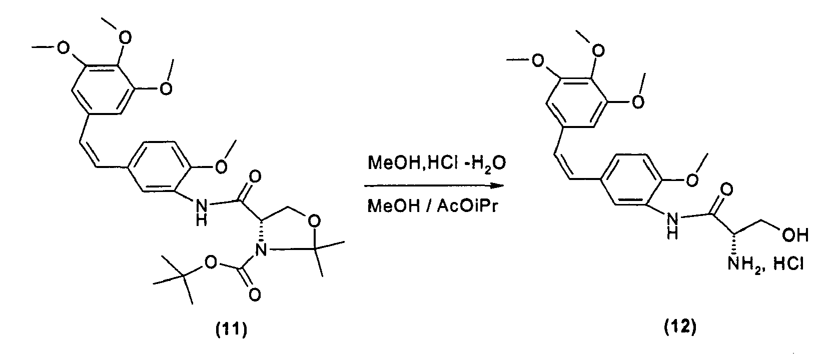

Dans un tricol de 1 litre muni d'une agitation mécanique, dun thermomètre, d'un tube Y, d'un réfrigérant surmonté d'un compte-bulles et d'un bain chauffant, on charge à 20°C 61,8 g de (11) en solution dans 54 ml de méthanol, on ajoute 150 ml d'acétate d'isopropyle, 99 ml de méthanol chlorhydrique à 2,3N et 8,2 ml d'eau distillée. On agite et on chauffe à 60°C pendant 3 heures. La solution refroidie à 40°C est clarifiée par filtration sur un verre fritté n°4 rincé par 40 ml de méthanol. On recharge le filtrat en tricol agité et on ajoute,194 ml d'acétate d'isopropyle, on réchauffe à 40°C, la solution est amorcée par 0,2 g de (12) puis on coule goutte à goutte en 1 heure, 194 ml d'acétate d'isopropyle, le milieu cristallise lentement pendant la coulée.

On laisse le milieu revenir à l'ambiante puis on refroidit et maintient la nuit à 5°C.

Le lendemain la bouillie est filtrée sur verre fritté, le gâteau essoré est lavé par 4 fois 50 ml dacétate d'isopropyle, essoré à fond puis séché à l'étuve à poids constant (35°C / 10 mmHg).

On obtient 28 g de **(12)** avec un rendement sur 2 étapes (couplage puis déprotections) de : 56 %, et un titre en CLHP NI > 98%.

Soit un rendement global tel quel, pour la synthèse réalisée selon le premier procédé voie V0 2 de : 30 %. [ **(12)** obtenu sur **(5)** engagé].

## Revendications

1. Procédé de préparation de combretastatine de formule: 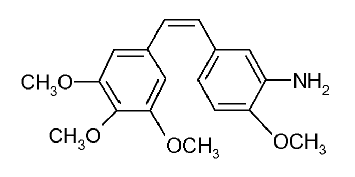 **caractérisé en ce que** :
• après la condensation de Wittig en présence de bromure ou chlorure du (3,4,5-triméthoxy-benzyle)-triphénylphosphonium et du 3-nitro-4-méthoxy benzaldéhyde ou du 3,4,5-triméthoxy-benzaldéhyde avec un sel de (4-méthoxy-3-nitro-benzyle)-triphénylphosphonium,
• on réalise la réduction du groupe nitro en présence de fer.

2. Procédé, selon la revendication 1 **caractérisé en ce que** la quantité de fer utilisé est en excès.

3. Procédé, selon la revendication 2 **caractérisé en ce que** cet excès est supérieur à 2 équivalents pour une mole de dérivé nitré de départ.

4. Procédé selon l'une des revendications 1 à 3 **caractérisé en ce que** la réaction est mise en oeuvre en présence d'une base choisie parmi le terbutylate de potassium, le téramylate de sodium, l'hydrure de sodium, le butyllithium, le LDA (Lithium Diisopropyl Amine), le méthylate de sodium, le carbonate de potassium ou les derivés alcalins des hexaméthyldisilanes.

5. Procédé selon l'une des revendications 1 à 4, **caractérisé en ce que** la réaction est mise en oeuvre en présence d'un solvant choisi parmi les éthers, les solvants aprotiques polaires, les alcools, les solvants aromatiques ou l'eau.

6. Procédé selon la revendication 5, **caractérisé en ce que** le solvant est choisi parmi le THF, l'acétonitrile, le NMP, le DMF, le DMSO.
